Europäisches Patentamt

European Patent Office

Office européen des brevets

(11) Veröffentlichungsnummer: **0 053 814**

**A1**

# (12) EUROPÄISCHE PATENTANMELDUNG

(21) Anmeldenummer: **81110149.2**

(22) Anmeldetag: **04.12.81**

(51) Int. Cl.³: **G 01 N 33/54**

(30) Priorität: **05.12.80 DE 3045872**

(43) Veröffentlichungstag der Anmeldung:
**16.06.82 Patentblatt 82/24**

(84) Benannte Vertragsstaaten:
**AT BE CH DE FR GB IT LI LU NL SE**

(71) Anmelder: **Niculescu, Vladimir Florin**
**Wilhelm-Raabe-Strasse 1**
**D-3004 Isernhagen(DE)**

(72) Erfinder: **Niculescu, Vladimir Florin**
**Wilhelm-Raabe-Strasse 1**
**D-3004 Isernhagen(DE)**

(74) Vertreter: **Leine, Sigurd, Dipl.-Ing. et al,**
**Dipl.-Ing. Sigurd Leine Dipl.-Phys. Dr. Norbert König**
**Patentanwälte Burckhardtstrasse 1**
**D-3000 Hannover 1(DE)**

(54) **Immunologisch-diagnostisches Verfahren zum Verbrauch und Nachweis heterophiler Antikörper im Serum.**

(57) Die Erfindung betrifft ein immunologisch-diagnostisches Verfahren zum Verbrauch und Nachweis heterophiler Antikörper im Serum. Um dieses Verfahren einfacher und schneller durchführbar, empfindlicher und spezifischer zu machen, wird die Agglutinationsfähigkeit aller oder eines bestimmten oder mehrerer bestimmter heterophiler Antikörper unterbunden, soweit zur Unterbindung der Agglutinationsfähigkeit gelöste oder lösbare Verbrauchsantigene eingesetzt werden. Die Verbrauchsantigene werden im Überschuß eingesetzt und mit konzentriertem Serum gemischt, um den Verbrauch an heterophilen Antikörpern in einer einzigen Reaktionsstufe zu erreichen. Zur Feststellung der Spezifität und Qualität der vorhandenen Agglutininmoleküle werden die heterophilen Agglutinine zu einer geometrischen Reihe Agglutinineinheiten austitriert und vorbestimmten Dosen Verbrauchsantigenen ausgesetzt. Vorzugsweise werden die Verbrauchsantigenlösungen mittels Dehydrierung in rekonstituierbare Trockenantigene umgewandelt.

EP 0 053 814 A1

LEINE & KÖNIG

PATENTANWÄLTE

0053814

Dipl.-Ing. Sigurd Leine · Dipl.-Phys. Dr. Norbert König

Burckhardtstraße 1          Telefon (0511) 62 30 05
D-3000 Hannover 1

Unser Zeichen          Datum
587/1 EPÜ    2. Dezember 1981

— 1 —

Vladimir Florin Niculescu

Immunologisch-diagnostisches Verfahren zum Verbrauch
und Nachweis heterophiler Antikörper im Serum.

Die Erfindung betrifft ein immunologisch-diagnostisches
Verfahren gemäß Oberbegriff des Anspruchs 1.

Heterophile Antikörper, die auch als heterophile Agglutinine bezeichnet werden, wurden 1932 von Paul und Bunnel
bei Patienten mit infektiöser Mononucleose (IM) beschrieben.
Ähnliche Agglutinine gegen Fremderythrozyten hat schon Davidsohn 1929 sowohl bei gesunden Personen als auch im Verlauf
der Serumkrankheit beobachtet. Später wurden heterophile
Antikörper bei etlichen Infektionskrankheiten nachgewiesen.
Eine chemische Ähnlichkeit zwischen Oberflächenantigenen
der als Testerythrozyten verwendeten Säugetier-Erythrozyten
und den Krankheitserregern wäre eine Erklärung der beobachteten Kreuzreaktionen.

Die meisten heterophilen Antikörper reagieren, wie bekannt, mit dem hitzeempfindlichen Forssman-Antigen der Erythrozyten (das Forssman-Antigen ist ein beinahe ubiquitäres
Antigen, das nicht nur an der Oberfläche von Erythrozyten,
sondern einer Vielzahl von Zellen vorkommt.) Die durch IM-Antikörper hervorgerufene Agglutination beruht jedoch auf ihrer Bindung an ein zweites hitzestabiles Antigen der Erythrozytenoberfläche. Lediglich die selten vorkommenden Agglutinine der Serumkrankheit sind bivalente Agglutine, mit Affinität zu den beiden oben erwähnten Antigenen.

Diese Erkenntnisse ermöglichten schließlich den Nachweis der infektiösen Mononucleose zu einer Zeit, als der Epstein-Barr-Virus als Erreger dieser Krankheit noch unbekannt war. Der daraus resultierende Test beruht auf der Diffetentialabsorption der heterophilen Antikörper. Es werden dazu zwei Absorptionspartikel verwendet, die jeweils nur eines der beiden oben erwähnten Antigene tragen, und zwar dienen Nierenteilchen vom Pferd oder Meerschweinchen als Absorptionspartikel für Forssman-Agglutinine, während Erythrozytenstroma vom Rind als Absorptionspartikel für IM-Agglutinine wirken. Ein Teil des Probenserums wird auf Nierenpartikel, ein anderer auf Erythrozytenstroma absorbiert. Kann das mit Nierenteilchen vermischte Serum weiterhin eine Agglutination von Testerythrozyten hervorrufen und bleibt eine solche Agglutination nach Absorption an Rindererythrozytenstroma aus, so gilt der Befund als positiv.

Tests, die auf dem bisherigen Verfahren beruhen, setzen die vollständige Absorption der heterophilen Antikörper durch das jeweilige Absorptionspartikel voraus. Dennoch kommt es in der Laborpraxis nicht selten vor, daß wegen Antigenarmut bzw. unvollständiger Absorption von Forssman Agglutininen positive IM-Befunde entstehen. Für diesen Fehler sind besonders IM-Objektträgertests anfällig. Deshalb empfehlen viele Autoren, positive Befunde solcher Schnelltests durch Titerbestimmungen im quantitativen Paul-Bunnel-Davidsohn Test zu überprüfen. Diese Testmethode ist aber zeitraubend und unhandlich, da von jedem Serum zwei Parallelproben anzusetzen sind, die absorbiert, zentrifugiert und dabei mehrfach pipettiert werden müssen.

Bei indirekten Hämagglutinationstests ist die vollständige Entfernung der heterophilen Agglutinine eine "conditio sine qua non", da künstlich mit Antigen beladene Testerythrozyten außerdem die bereits erwähnten Oberflächenantigene vom Typ Forssman und IM besitzen. Eine Vorabsorption des Serums an Kontrollerythrozyten ist erforderlich, wobei bekanntermaßen keine vollständige Bindung der heterophilen Agglutinine erfolgt.

Die der Erfindung zugrundeliegende Aufgabe besteht nun darin, ein Verfahren gemäß Oberbegriff des Anspruchs 1 anzugeben, das die obigen Nachteile nicht aufweist und das insbesondere einfacher und schneller durchführbar, empfindlicher und spezifischer ist.

Die der Erfindung zugrundeliegende Aufgabe wird durch die im Kennzeichen des Anspruchs 1 angegebenen Verfahrensmerkmale gelöst.

Das erfindungsgemäße Verfahren weist folgende Vorteile auf :

Es ist durch Verzicht auf Absorptions- und Zentrifugationsvorgänge handlich sowie zeit- und materialsparend. Das erfindungsgemäße Verfahren besitzt gegenüber herkömmlichen Tests optimale Spezifität und Aussagekraft.

Vorteilhafte und zweckmäßige weitere Ausgestaltungen der erfindungsgemäßen Lösung sin d in den weiteren Unteransprüchen gekennzeichnet.

Gemäß Anspruch 2 werden erfindungsgemäß zur Unterbindung der Agglutinationsfähigkeit heterophiler Antikörper gelöste oder lösbare Verbrauchsantigene eingesetzt. Sowohl die aus Verbrauchsantigenen und Agglutininen bestehenden Komplexe als auch die ungebundenen Verbrauchsantigene stören den weiteren Verlauf des erfindungsgemäßen Verfahrens nicht und müssen deshalb aus der Probe nicht entfernt werden.

Die oben erwähnten Vorteile des erfindungsgemäßen Verfahrens sind bezeichnend für alle aus dem Oberbegriff des Anspruchs 1 abgeleiteten Tests. Sie gelten sowohl für Tests, die Verbrauchsantigene im Überschuß einsetzen, um den vollständigen Verbrauch eines bestimmten oder mehrerer bestimmter heterophiler Antikörper in einem einzigen Testabschnitt zu erreichen, als auch für Tests, die heterophile Antikörper in mehreren Testabschnitten (Reaktionsstufen) ausschalten und dabei geringere Mengen Verbrauchsantigene benötigen.

Die zuletzt genannten Tests, die sich einer vorbestimmten Dosis Verbrauchsantigene bedienen, verbrauchen einen großen Teil der heterophilen Antikörper in einer ersten Reaktionsstufe, nachdem das Serum konzentriert oder vorverdünnt mit einem oder mehreren Verbrauchsantigenen vermischt wird. Der Anteil heterophiler Antikörper eines bestimmten Typs oder mehrerer bestimmter Typen, die in der ersten Reaktionsstufe verbraucht werden, ist sowohl von der Dichte als auch von der Affinität (Qualität) der Antikörpermoleküle zu den Verbrauchsantigenen bestimmt.

Nach erfolgter Reaktion wird eine <u>zweite Reaktionsstufe</u> eingeleitet, indem das vorliegende Gemisch in eine Verdünnungsreihe mit einem oder mehreren Verbrauchsantigenen überführt wird. Die in der ersten Reaktionsstufe nicht verbrauchten heterophilen Agglutinine werden zu einer geometrischen Reihe von Agglutinationseinheiten (AE) austitriert. Der Verbrauch heterophiler Agglutinine ist in der zweiten Reaktionsstufe ausschließlich von der Qualität der Antikörpermoleküle abhängig. Im Gegensatz zu herkömmlichen Tests ermöglicht das erfindungsgemäße Verfahren somit auch Aussagen über die Qualität der heterophilen Antikörper.

Es sei insbesondere noch auf die Lehre gemäß Anspruch 15 verwiesen, durch die Verbrauchsantigen-Lösungen sozusagen konserviert werden, wodurch sie gebrauchsfertig und für lange Zeit lagerfähig sind.

Ferner sei noch auf den Anspruch 17 verwiesen, der einen vorgefertigten, gebrauchsfähig präparierten Träger zur Durchführung des immunologisch-serologischen Diagnostik-Verfahrens betrifft. Die Herstellung solcher gebrauchsfertiger Testträger wird erst durch die vorliegende Erfindung ermöglicht.

– 6 –

Die Erfindung soll nachfolgend an einigen Beispielen näher erläutert werden.

Beispiel 1 :

Herstellung von Verbrauchsantigenen

Verbrauchsantigene werden mit Hilfe des nicht ionogenen Detergens Triton X-100 ($C_{34}H_{62}O_{11}$, octylphenoldecaäthylen-glycoläther) in Lösung gebracht. Antigen-haltiges Gewebematerial wird über Nacht unter Rühren gepuffertem 1%-igem Triton X-100 ausgesetzt. Das quantitative Verhältnis zwischen Ausgangsmaterial und dem Lösungsvermittler (1%-iges Triton X-100) ist 1:3 (v/v). Eine etwaige Vorbehandlung des Gewebematerials mit 5% Formalin ist zulässig.

Nach der Gewebeauflösung wird das Gemisch zentrifugiert und der Anteil des Lösungsvermittlers Triton X-100 auf eine für Testerythrozyten unschädliche Konzentration verringert, indem man z.B. den Überstand mit neuem Ausgangsmaterial behandelt. Der Reinheitsgrad bzw. die Unschädlichkeit der Verbrauchsantigen-Lösung wird an Testerythrozyten überprüft, während die quantitative Ausbeute an bekannten agglutininhaltigen Seren festgestellt wird. Die Aktivität der gewonnenen Verbrauchsantigene wird in Verbrauchseinheiten (VE) erfaßt. Eine VE entspricht jener Menge an Verbrauchsantigen, die in der Lage ist, eine Agglutinineinheit (AE) auszuschalten. Die Einstellung der Aktivität der Endprodukte erfolgt, nachdem die optimale Dosis der Verbrauchsantigene gegenüber Forssman- und IM-Agglutininen ermittelt worden ist.

Die gereinigten Verbrauchsantigene können über Monate hinaus bei $4^{O}C$ ohne Additiva aufbewahrt werden. Einfrieren bei $-20^{O}C$ hat sich als ungeeignet erwiesen.

Nach der Endeinstellung können die Verbrauchsantigene auf Mikrotiterplatten oder Objektträger aufgebracht und bei $+4^{O}C$ dehydriert werden. Auch auf Träger geladene dehydrierte Verbrauchsantigene können über Monate hinaus aufbewahrt werden.

Beispiel 2 :

Nachweis von IM-Agglutininen an vorbeladenen Mikrotiterplatten

Eine mit Verbrauchsantigenen beladene Mikrotiterplatte mit in einem vorgegebenen Raster angeordneten Vertiefungen (z.B. A bis H / 1 bis 12-Raster) ist für zwei IM-Bestimmungen mit Titerangabe vorgesehen. Für jede Bestimmung werden vier waagerechte Reihen (z.B. A bis D) verwendet.

Die Platte ist wie folgt beladen :

(i) die Vertiefungen $B_1$, $C_{1-12}$ und $D_1$ enthalten dehydriertes Verbrauchsantigen vom Typ Forssman,

(ii) die Vertiefungen $D_{2-12}$ enthalten dehydriertes Verbrauchsantigen vom Typ IM,

(iii) alle anderen Vertiefungen enthalten Puffersalze.

Vor Beginn des Tests werden die Inhalte der Plattenvertiefungen mit destilliertem Wasser rekonstituiert.

Der Verbrauch heterophiler Agglutinine findet in zwei Reaktionsstufen statt. Zuerst werden die antigenhaltigen

Vertiefungen $B_1$, $C_1$ und $D_1$ mit gleichen Mengen Probeserum versehen; die gleiche Menge Serum wird auch in die antigenfreie Vertiefung $A_1$ gegeben. Nach einer bestimmten ersten Reaktionszeit werden die Gemische der ersten Reaktionsstufe in die jeweiligen Verdünnungsreihen (geometrische Reihe Agglutinineinheiten) überführt ($A_2$ bis $A_{12}$, $B_2$ bis $B_{12}$, $C_2$ bis $C_{12}$, $D_2$ bis $D_{12}$). Dabei werden in den Reihen C und D die noch nicht verbrauchten Forssman- bzw. IM-Agglutinine mit Verbrauchsantigen austitriert.

Nach einer zweiten Reaktionszeit werden Testerythrozyten vom Schaf zugefügt. Bei Endvolumina von $100\,\mu$l beträgt die Konzentration der Erythrozyten jeweils 0,2%.

Das Ergebnis wird nach weiteren 90 Minuten abgelesen. Kommt es in den Testreihen D zu einem Titerrücklauf von mindestens zwei Stufen gegenüber der Reihe C, so ergibt sich für das Probenserum ein positiver IM-Befund. Der Titer der heterophilen IM-Antikörper ist in der Reihe C abzulesen.

Beispiel 3 :

Vorbeladene Mikrotiterplatte für den Verbrauch heterophiler Antikörper im indirekten Hämagglutinationstest

Eine mit Verbrauchsantigenen beladene Mikrotiterplatte ist für sechs Bestimmungen im indirekten Hämagglutinationstest vorgesehen. Für jede Bestimmung werden zwei der insgesamt zwölf waagerechten Reihen des Vertiefungsrasters der Mikrotiterplatte mit je acht Vertiefungen verwendet.

Jede Vertiefung enthält rekonstituierbare Verbrauchsantigene vom Typ Forssman und IM. Die Antigendosis ist in Verbrauchseinheiten (VE) angegeben.

Vor Beginn des Tests werden die Inhalte der Plattenvertiefungen mit destilliertem Wasser rekonstituiert.

Der Verbrauch heterophiler Agglutinine findet in zwei Reaktionsstufen statt. Zuerst werden mit gleichen Mengen konzentriertem Probenserum zwei H-Vertiefungen (z.B. $H_1$ und $H_2$) versehen. Nach einer vorgesehenen ersten Reaktionszeit werden die Gemische der ersten Reaktionsstufe in die jeweiligen Verdünnungsreihen überführt ($F_1$ bis $A_1$ und $F_2$ bis $A_2$). Nach einer zweiten Reaktionszeit werden die Testerythrozyten zugefügt; die erste Verdünnungsreihe erhält dabei antigenfreie Kontrollerythrozyten, während die zweite Verdünnungsreihe mit Testantigen beladene Erythrozyten erhält.

Die Ergebnisse des Tests werden nach Ablauf einer für die indirekte Hämagglutination vorgesehene Testzeit abgelesen. Kommt es in der zweiten Testreihe zu einem Titeranstieg von mindestens zwei Titerstufen gegenüber der ersten Testreihe, so ergibt sich für das Testserum ein positiver Befund. Der Titer des Probeserums ist in der zweiten Testreihe abzulesen.

Beispiel 4 :

IM-Schnelltest auf vorgeladenen Objektträgern

Für eine IM-Schnellbestimmung sind zwei Vertiefungen (Felder) eines Objektträgers vorgesehen, die einen Überschuß an dehydrierten Verbrauchsantigenen vom Typ Forssman (Feld A) bzw. vom Typ IM (Feld B) enthalten.

Unmittelbar vor Beginn des Tests werden die Verbrauchsantigene mit destilliertem Wasser rekonstituiert. Gleiche Mengen Probenserum werden den rekonstituierten Verbrauchsantigenen zugefügt. Der Verbrauch der heterophilen Agglutinine wird durch Umrühren des Gemisches eingeleitet. Nach erfolgter Reaktionszeit erhält jedes Feld gleichermaßen Indikatorerythrozyten.

Das Ergebnis wird nach ca. 1 Minute abgelesen. Ein positiver Befund liegt vor, wenn lediglich die Erythrozyten im Feld A agglutinieren.

Dipl.-Ing. Sigurd Leine · Dipl.-Phys. Dr. Norbert König

Burckhardtstraße 1       Telefon (0511) 62 30 05
D-3000 Hannover 1

Vladimir Florin Niculescu

Unser Zeichen        Datum

587/1 EPÜ      2. Dezember 1981

P a t e n t a n s p r ü c h e

1. Immunologisch-diagnostisches Verfahren zum Verbrauch und Nachweis heterophiler Antikörper (Agglutinine) im Serum,

dadurch gekennzeichnet, daß die Agglutinationsfähigkeit aller oder eines bestimmten oder mehrerer bestimmter heterophiler Antikörper unterbunden wird.

2. Verfahren nach Anspruch 1, dadurch gekennzeichnet, daß zur Unterbindung der Agglutinationsfähigkeit heterophiler Antikörper gelöste oder lösbare Verbrauchsantigene eingesetzt werden.

3. Verfahren nach den Ansprüchen 1 und 2, dadurch gekennzeichnet, daß zum vollständigen Verbrauch aller oder eines bestimmten oder mehrerer bestimmter heterophiler Antikörper Verbrauchsantigene im Überschuß verwendet und mit konzentriertem Serum gemischt werden, wodurch der Verbrauch heterophiler Antikörper in einer einzigen Reaktionsstufe erfolgt.

Dr.K./H.

4. Verfahren nach den Ansprüchen 1 und 2, dadurch gekennzeichnet, daß zum anteiligen Verbrauch eines bestimmten oder mehrerer bestimmter heterophiler Antikörper eine vorbestimmte Dosis Verbrauchsantigen verwendet und mit konzentriertem bzw. schwach verdünntem Probeserum in einer ersten Reaktionsstufe gemischt wird, wobei der Anteil ausgeschalteter heterophiler Agglutinine von der Affinität (Qualität) der Agglutininmoleküle zu den Verbrauchsantigenen und der Dichte (Konzentration) der Agglutininmoleküle im Serum abhängt.

5. Verfahren nach Anspruch 4, dadurch gekennzeichnet, daß die noch nicht verbrauchten heterophilen Agglutinine zu einer geometrischen Reihe Agglutinineinheiten austitriert und in einer anschließenden zweiten Reaktionsstufe vorbestimmten Dosen Verbrauchsantigenen ausgesetzt werden, wodurch Spezifität und Qualität der vorhandenen Agglutininmoleküle feststellbar ist.

6. Verfahren nach den Ansprüchen 1 und 2, dadurch gekennzeichnet, daß die heterophilen Agglutinine zu einer geometrischen Reihe Agglutinineinheiten austitriert und vorbestimmten Dosen Verbrauchsantigenen ausgesetzt werden, wodurch Spezifität und Qualität der vorhandenen Agglutininmoleküle feststellbar ist.

7. Verfahren zur Herstellung der Verbrauchsantigene nach Anspruch 2, dadurch gekennzeichnet, daß Gewebeteilchen, die solche Verbrauchsantigene tragen, mit Lösungsmitteln,
Lösungsvermittlern, Detergentien usw. behandelt werden,
wodurch die Verbrauchsantigene in Lösung gehen.

8. Verfahren nach Anspruch 3, <u>dadurch gekennzeichnet</u>, daß
der Anteil der Lösungsmittel, Lösungsvermittler, Detergentien usw. in der Verbrauchsantigen-Lösung auf eine für
die Durchführung immunologischer Tests subkritische Konzentration verringert (eingestellt) wird, so daß eine Hämolyse
innerhalb der vorgesehenen Testzeit nicht auftritt.

9. Verfahren nach Anspruch 7, <u>dadurch gekennzeichnet</u>, daß
die Lösungsmittel, Lösungsvermittler und Detergentien usw.
aus der wäßrigen Verbrauchsantigen-Lösung entfernt werden.

1o. Verfahren nach den Ansprüchen 7 und 8, <u>dadurch gekenn-
zeichnet</u>, daß die Lösungsmittel, Lösungsvermittler, Detergentien usw. durch Zugabe weiterer Verbrauchsantigene tragender Gewebeteilchen und anschließende Zentrifugation entfernt werden.

11. Verfahren nach den Ansprüchen 2, 7, 8, 9oder 1o,
<u>dadurch gekennzeichnet</u>, daß Verbrauchsantigen-Lösungen
mittels Dehydrierung in rekonstituierbare Trockenantigene
umgewandelt werden.

12. Verfahren nach Anspruch 11, dadurch gekennzeichnet, daß die Dehydrierung nach dem Auftragen der Verbrauchsantigen-Lösungen auf Träger für die immunologische Diagnostik erfolgt.

13. Verfahren nach den Ansprüchen 11 oder 12, dadurch gekennzeichnet, daß die Dehydrierung durch Trocknung im Vakuum erfolgt.

14. Verfahren nach den Ansprüchen 11 oder 12, dadurch gekennzeichnet, daß die Dehydrierung durch Gefriertrocknung erfolgt.

15. Verfahren zur Herstellung einer lagerfähigen gebrauchsfertigen Verbrauchsantigen-Lösung, dadurch gekennzeichnet, daß Geweberteilchen, die die Verbrauchsantigene tragen, mit diese Verbrauchsantigene in Lösung bringenden Lösungsmitteln, Lösungsvermittlern, Detergentien usw. behandelt werden und daß in der erhaltenen Lösung die Lösungsmittel, Lösungsvermittler oder Detergentien usw. auf eine für immunologische Tests subkritische Konzentration gebracht werden.

16. Verfahren nach einem der vorhergehenden Ansprüche, dadurch gekennzeichnet, daß als Lösungsmittel, Lösungsvermittler oder Detergentien nichtionogene Detertentien, bei- spielsweise $C_{34}H_{62}O_{11}$, octylphenoldecaäthylenglycoläther, verwendet werden.

17. Träger für die immunologisch-serologische Diagnostik mit einer Testsubstanz nach einem der vorhergehenden Ansprüche oder einer anderen Testsubstanz, die gelöst aufgebracht und auf dem Träger in eine rekonstituierbare Trockensubstanz überführt ist.

\

0053814

Europäisches
Patentamt          **EUROPÄISCHER RECHERCHENBERICHT**          Nummer der Anmeldung

EP 81 11 0149

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | **G 01 N 33/54** |
| X | <u>US - A - 4 139 606</u> (ZICHIS J.)<br>* Seite 4, Spalte 9; Anspruch 4 *<br><br>-- | 1,2,3, 4,5,6 | |
| A | <u>DE - A - 2 847 877</u> (BEHRINGWERKE A.G.) | | |
| A | <u>DE - A - 1 920 866</u> (ORTHO PHARMA-CEUTICAL CORPORATION) | | |
| A | CHEMICAL ABSTRACTS, Band 86, Nr. 15, April 1977, Seite 331, Zusammenfassung 104280h COLUMBUS, OHIO, U.S.A. M.A. FLETCHER et al.: "Immunochemical Studies of Infectious Mononucleosis. VI. A Radioimmunoassay for the Detection of Infectious Mononucleosis Heterophile Antibody and Antigen"<br>& IMMUNOL METHODS 1977, 14(1),51-8<br>* Zusammenfassung *<br><br>---- | 17 | **RECHERCHIERTE SACHGEBIETE (Int. Cl.³)**<br><br>G 01 N |

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

| X | Der vorliegende Recherchenbericht wurde für alle Patentansprüche erstellt. |
|---|---|

| Recherchenort | Abschlußdatum der Recherche | Prüfer |
|---|---|---|
| Den Haag | 18-03-1982 | OSBORNE |

EPA form 1503.1   06.78